# EUROPEAN PATENT SPECIFICATION

(11) **EP 3 839 070 B1**
(45) Date of publication and mention of the grant of the patent: **22.07.2026**
(21) Application number: 19849884.2
(22) Date of filing: 08.08.2019
(51) Int. Cl.: C12Q 1/6886, C12N 15/11

(54) **DNA METHYLATION-RELATED MARKER FOR DIAGNOSING TUMOR, AND APPLICATION THEREOF**
MARKER IM ZUSAMMENHANG MIT DER DNA-METHYLIERUNG ZUR DIAGNOSE VON TUMOREN UND ANWENDUNG DAVON
MARQUEUR LIÉ À LA MÉTHYLATION DE L'ADN POUR DIAGNOSTIQUER UNE TUMEUR, ET SON APPLICATION

(30) Priority: 16.08.2018 CN 201810935933
(43) Date of publication of application: 23.06.2021
(73) Proprietor: Shanghai Public Health Clinical Center, Shanghai 201058 (CN)
(72) Inventor: YU, Wenqiang, Shanghai 200032 (CN); DONG, Shihua, Shanghai 200032 (CN)
(74) Representative: Oryon NV
(86) International application number: PCT/CN2019/099722
(87) International publication number: WO 2020/034888

(56) References cited:
- WO-A1-2015/155660
- WO-A1-2016/115354
- WO-A1-2018/111935
- WO-A2-2014/173905
- WO-A2-2015/191780
- CN-A- 104 450 872
- ANONYMOUS: "GS_NUC_ALERT:WO2015191780.120079", 17 December 2015 (2015-12-17), pages 1 - 1, XP055910733, Retrieved from the Internet <URL:http://ibis.internal.epo.org/exam/dbfetch.jsp?id=GS_NUC_ALERT:WO2015191780.120079> [retrieved on 20220408]
- ANONYMOUS: "Homo sapiens histone H3 (HIST1H3C) gene, complete cds - Nucleotide - NCBI - AF531276", 23 October 2002 (2002-10-23), XP055910846, Retrieved from the Internet <URL:https://www.ncbi.nlm.nih.gov/nucleotide/AF531276.1?report=genbank&log$=nuclalign&blast_rank=1&RID=5034X8G901R> [retrieved on 20220408]
- ANONYMOUS: "GSN:BCF53369", 3 December 2015 (2015-12-03), XP055910977, Retrieved from the Internet <URL:http://ibis.internal.epo.org/exam/dbfetch.jsp?id=GSN:BCF53369> [retrieved on 20220408]
- ANONYMOUS: "Homo sapiens histone H2B (HIST1H2BE) gene, complete cds - Nucleotide - AF531288", 23 October 2002 (2002-10-23), XP055911005, Retrieved from the Internet <URL:https://www.ncbi.nlm.nih.gov/nucleotide/AF531288.1?report=genbank&log$=nuclalign&blast_rank=3&RID=50GU9BG2016> [retrieved on 20220408]
- NAYAK SHWETA R ET AL: "A Role for Histone H2B Variants in Endocrine-Resistant Breast Cancer", HORMONES AND CANCER, SPRINGER NEW YORK LLC, US, vol. 6, no. 5, 26 June 2015 (2015-06-26), pages 214 - 224, XP035789229, ISSN: 1868-8497, [retrieved on 20150626], DOI: 10.1007/S12672-015-0230-5
- NAYAK SHWETA R ET AL: "A Role for Histone H2B Variants in Endocrine-Resistant Breast Cancer", HORMONES AND CANCER, vol. 6, no. 5-6, 26 June 2015 (2015-06-26), pages 214 - 224, XP037322548, ISSN: 1868-8497, DOI: 10.1007/S12672-015-0230-5
- DATABASE Nucleotide 23 October 2002 (2002-10-23), "Homo sapiens clone HIST1H4F histone H4 gene, complete cds", XP055688586, retrieved from ncbi Database accession no. AY128659.1
- DATABASE Nucleotide 24 January 2013 (2013-01-24), "Human DNA sequence from clone RP1-221C16 on chromosome 6, complete sequence", XP055688600, retrieved from ncbi Database accession no. AL353759.8
- DATABASE Nucleotide 23 October 2002 (2002-10-23), "Homo sapiens histone H3 (HIST1H3C) gene, complete cds", XP055688603, retrieved from ncbi Database accession no. AF531276.1
- DATABASE Nucleotide 23 October 2002 (2002-10-23), "Homo sapiens clone HIST1H4I histone H4 gene, complete cds", XP055688604, retrieved from ncbi Database accession no. AY128662.1
- GEZER, U. ET AL.: "Histone Methylation Marks on Circulating Nucleosomes as Novel Blood- Based Biomarker in Colorectal Cancer", INTERNATIONAL JOURNAL OF MOLECULAR SCIENCES, vol. 16, no. 12, 11 December 2015 (2015-12-11), pages 29654 - 29662, XP055688577
- ZHANG, LIANHAI ET AL.: "Research on Prevention and Treatment of Gastric Cancer: S100A6 May Serve as a Biomarker for Gastric Cancer", BASIC ONCOLOGY - THE 18TH BRANCH OF THE 13TH CHINA ASSOCIATION FOR SCIENCE AND TECHNOLOGY ANNUAL CONFERENCE; 2011.09.21-22, 21 September 2011 (2011-09-21), pages 90 - 92, XP009526431

## Description

### FIELD OF DISCLOSURE

The disclosure relates to the field of diagnosis. More specifically, the disclosure relates to a DNA methylation related marker for diagnosing of tumor and application thereof.

### BACKGROUND OF DISCLOSURE

With the development of tumor research, more and more evidences in the art show that small changes in epigenetic regulation have an important role in tumor.

Epigenetics is a subject that studies that the heritable change of gene function without a change of DNA sequence, which eventually leads to the change of phenotype. Epigenetics mainly includes DNA methylation, histone modification, microRNA level changes and other biochemical processes. DNA methylation refers to the process of transferring methyl from S-adenosylmethionine (methyl donor) to specific bases under the catalysis of DNA methyltransferase. DNA methylation can occur at N-6 of adenine, N-4 of cytosine, N-7 of guanine or C-5 of cytosine. However, DNA methylation in mammals mainly occurs at the C of 5'-CpG-3', which results in 5-methylcytosine (5mC).

More than 98% of CpG dinucleotides are scattered in repetitive sequences with transcription dependent transposition potential. In normal cells, these CPGs are hypermethylated/transcriptionally silenced. While in tumor cells, they undergo extensive demethylation, leading to transcription of repetitive sequences, activation of transposons, high genomic instability and enhanced transcription of proto-oncogenes. The remaining 2% of the total CpGs was densely distributed in smaller areas (CpG islands). About 40% - 50% of the gene promoter regions have CpG islands, suggesting that DNA methylation may be involved in the transcriptional regulation of these genes. In some tumors, these CpG islands, which were hypomethylated in normal cells, will be hypermethylated, leading to inactivation of gene transcription. The affected genes include DNA repair genes, cell-cycle control genes and anti-apoptosis genes.

In view of the high false positive and false negative of tumor markers commonly used in clinic, such as alpha fetoprotein (AFP), it is urgent to develop new markers in this art. It is a new way to study tumor biomarkers by abnormal DNA methylation profile of tumor cells. However, it needs a lot of research and long-term gene identification and comparison of a large number of patients in order to find the abnormal methylation profile that are really related to a tumor.

### SUMMARY OF DISCLOSURE

The object of the disclosure is to provide DNA methylation related markers for diagnosing tumor and application thereof.

The first aspect of the present disclosure provides an isolated polynucleotide, comprising:
(a) the polynucleotide with the nucleotide sequence shown in SEQ ID NO: 1;
(b) the polynucleotide with the nucleotide sequence shown in SEQ ID NO: 3;
(c) the polynucleotide with the nucleotide sequence shown in SEQ ID NO: 5;
(d) the polynucleotide with the nucleotide sequence shown in SEQ ID NO: 7;
(e) a fragment of the polynucleotide of (a)-(d), having at least one (such as 2-40, more specifically 3, 5, 8, 10, 15, 20, 25, 30, 35) CpG site with modification; and/or
(f) a nucleic acid (antisense strand) complementary to the polynucleotide or fragment of (a)-(e).

In a preferable embodiment, the modification includes 5-methylation (5mC), 5-hydroxymethylation (5hmC), 5-aldehyde methylation (5-fC) or 5-carboxymethylation (5-caC).

Another aspect of the disclosure provides an isolated polynucleotide, which is converted from the polynucleotide, and as compared with any sequence of the above (a)-(e), its cytosine C of the CpG site(s) with modification is unchanged, and the unmodified cytosine is converted into T(U).

In a preferable embodiment, it is converted from the polynucleotide corresponding to the above (a)-(e) by bisulfite treatment.

In another preferable embodiment, the polynucleotide comprises:
(g) the polynucleotide with the nucleotide sequence shown in SEQ ID NO: 2;
(h) the polynucleotide with the nucleotide sequence shown in SEQ ID NO: 4;
(i) the polynucleotide with the nucleotide sequence shown in SEQ ID NO: 6;
(j) the polynucleotide with the nucleotide sequence shown in SEQ ID NO: 8;
(k) a fragment of the polynucleotide of (g)-(j), having at least one (such as 2-40, more specifically 3, 5, 8, 10, 15, 20, 25, 30, 35) CpG site with modification.

Another aspect of the disclosure provides a use of any polynucleotide described above in manufacture of a tumor detection agent or kit.

In a preferable embodiment, the tumors include: digestive system tumors such as esophageal cancer, gastric cancer, colorectal cancer, liver cancer, pancreatic cancer, bile duct and gallbladder cancer; respiratory system tumors such as lung cancer, pleuroma; hematologic cancers such as leukemia, lymphoma, multiple myeloma; gynecological and reproductive system tumors such as breast cancer, ovarian cancer, cervical cancer, vulvar cancer, testicular cancer, prostate cancer, penile cancer; nervous system tumors such as glioma, neuroblastoma, meningioma; head and neck tumors such as oral cancer, tongue cancer, laryngeal cancer, nasopharyngeal cancer; urinary system tumors such as kidney cancer, bladder cancer, skin and other systems tumors such as skin cancer, melanoma, osteosarcoma, liposarcoma, thyroid cancer.

Another aspect of the disclosure provides a method of preparing a tumor detection agent, including: providing any polynucleotide (such as 1, 2, 3, or 4) described above, designing a detection agent for specifically detecting a target sequence which is the full length or fragment of the polynucleotide; wherein, the target sequence has at least one (such as 2-40, more specifically, 3, 5, 8, 10, 15, 20, 25, 30, 35) modified CpG site; preferably, the detection agent includes (but is not limited to) a primer, a probe.

Another aspect of the disclosure provides an agent or a combination of agents which specifically detect a target sequence, which is the full length or fragment of any of the polynucleotides described above and has at least one (such as 2-40, more specifically, 3, 5, 8, 10, 15, 20, 25, 30, 35) modified CpG site; preferably, the detection agent includes (but is not limited to) a primer, a probe.

In a preferable embodiment, the polynucleotide is the polynucleotide of the nucleotide sequence shown in SEQ ID NO: 1, and the target sequence contains the fragment of residues 240-296 of SEQ ID NO: 1; preferably, the primer is as shown in SEQ ID NO: 9-12.
In another preferable embodiment, the polynucleotide is the polynucleotide of the sequence shown in SEQ ID NO: 3, and the target sequence contains the fragment of residues 279-323 of SEQ ID NO:3; preferably, the primer is as shown in SEQ ID NO: 12-15; or
In another preferable embodiment, the polynucleotide is the polynucleotide of the sequence shown in SEQ ID NO: 5, and the target sequence contains the fragment of residues 186-235 of SEQ ID NO:5; preferably, the primer is as shown in SEQ ID NO: 12, 16-18; or
In another preferable embodiment, the polynucleotide is the polynucleotide of the sequence shown in SEQ ID NO: 7, and the target sequence contains the fragment of residues 164-198 of SEQ ID NO:7; preferably, the primer is as shown in SEQ ID NO: 12, 19-21.

Another aspect of the disclosure provides the use of the agent or combination of agents in the manufacture of a kit for detecting tumors; preferably, the tumors comprise: digestive system tumors such as esophageal cancer, gastric cancer, colorectal cancer, liver cancer, pancreatic cancer, bile duct and gallbladder cancer; respiratory system tumors such as lung cancer, pleuroma; hematologic cancers such as leukemia, lymphoma, multiple myeloma; gynecological and reproductive system tumors such as breast cancer, ovarian cancer, cervical cancer, vulvar cancer, testicular cancer, prostate cancer, penile cancer; nervous system tumors such as glioma, neuroblastoma, meningioma; head and neck tumors such as oral cancer, tongue cancer, laryngeal cancer, nasopharyngeal cancer; urinary system tumors such as kidney cancer, bladder cancer, skin and other systems tumors such as skin cancer, melanoma, osteosarcoma, liposarcoma, thyroid cancer.

Another aspect of the present disclosure provides a detection kit, comprising
container(s) and the agent or combination of agents described above in the container(s); preferably, each agent is located in an independent container.

In a preferable embodiment, the kit also includes: bisulfite, DNA purification agent, DNA extraction agent, PCR amplification agent and/or instruction for use (indicating operation steps of the detection and a result judgment standard).

In another aspect of the disclosure, a method of detecting the methylation profile of the polynucleotide or a fragment thereof in a sample *in vitro* is provided, comprising:
(i) providing the sample and extracting DNA;
(1) treating the sample to be detected to convert the unmodified cytosine into uracil; preferably, the modification includes 5-methylation (5mC), 5-hydroxymethylation (5hmC), 5-aldehyde methylation or 5-carboxymethylation; preferably, treating the DNA of step (i) with bisulfite;
(iii) analyzing the modification of the polynucleotide or a fragment thereof in the genomic DNA treated by step (ii).

In a preferable embodiment, the abnormal methylation profile is the high level of methylation of C in CPG(s) of the polynucleotide.

In another preferable embodiment, the methylation profile detecting method is not for the purpose of directly obtaining the diagnosis result of a disease, or is not a diagnostic method.

In another preferable embodiment, in step (3), the analysis methods include (but are not limited to): pyrosequencing, bisulfite conversion sequencing, qPCR, second generation sequencing, whole genome methylation sequencing, DNA enrichment detection, simplified bisulfite sequencing technology, HPLC, or their combination.

Other aspects of the disclosure will be apparent to those skilled in the art based on the disclosure herein.

### BRIEF DESCRIPTION OF DRAWINGS

Figure 1. Detection results of CTSM-4F for colorectal cancer.
Figure 2. Detection results of CTSM-4F for liver cancer.
Figure 3. Detection results of CTSM-4F for head and neck tumor.
Figure 4. Detection results of CTSM-4F for lung cancer.
Figure 5. Detection results of CTSM-4F for breast cancer.
Figure 6. Detection results of CTSM-2BE for head and neck cancer.
Figure 7. Detection results of CTSM-2BE for lung cancer.
Figure 8. Detection results of CTSM-2BE for pancreatic cancer.
Figure 9. Detection results of CTSM-3C for colorectal cancer.
Figure 10. Detection results of CTSM-3C for head and neck cancer.
Figure 11. Detection results of CTSM-3C for lung cancer.
Figure 12. Detection results of CTSM-4I for breast cancer.
Figure 13. Detection results of CTSM-4I for colorectal cancer.
Figure 14. Detection results of CTSM-4I for head and neck cancer.
Figure 15. Detection results of CTSM-4I for lung cancer.

### DETAILED DESCRIPTION

The inventor is committed to the research of tumor markers. After extensive research and screening, gene sequence regions with abnormal DNA methylation in the genome have been identified and named as tumor markers CTSM-4F, CTSM-2BE, CTSM-3C and CTSM-4I. Clinical studies show that the methylation status of these regions is significantly different between a tumor tissue and a non-tumor tissue, i.e., their CpG(s) was hypermethylated. Therefore, these genes are tumor markers and can be used as the basis of designing tumor diagnostic agents.

### Term

As used herein, "isolated" refers to a material separated from its original environment (if the material is a natural material, the original environment is the natural environment). For example, in living cells, polynucleotides and polypeptides in their natural state are not isolated or purified, but the same polynucleotides or polypeptides will be isolated ones if they are separated from other substances existed in the natural state.

As used herein, "sample" includes substances suitable for DNA methylation detection obtained from any individual or isolated tissue, cell or body fluid.

As used herein, "hypermethylation" refers to high level of methylation, hydroxymethylation, aldehyde methylation or carboxymethylation of CpG in a gene sequence. For example, in the case of methylation specific PCR (MSP), if the PCR reaction with methylation specific primers has positive PCR results, the DNA (gene) region of interest is in hypermethylation state. For another example, in the case of real-time quantitative methylation specific PCR, hypermethylation can be determined based on statistic difference of the methylation status value as compared with the control sample.

As used herein, the tumors include but are not limited to: digestive system tumors such as esophageal cancer, gastric cancer, colorectal cancer, liver cancer, pancreatic cancer, bile duct and gallbladder cancer; respiratory system tumors such as lung cancer, pleuroma; hematologic cancers such as leukemia, lymphoma, multiple myeloma; gynecological and reproductive system tumors such as breast cancer, ovarian cancer, cervical cancer, vulvar cancer, testicular cancer, prostate cancer, penile cancer; nervous system tumors such as glioma, neuroblastoma, meningioma; head and neck tumors such as oral cancer, tongue cancer, laryngeal cancer, nasopharyngeal cancer; urinary system tumors such as kidney cancer, bladder cancer, skin and other systems tumors such as skin cancer, melanoma, osteosarcoma, liposarcoma, thyroid cancer.

### Gene marker

In order to find a useful target for tumor diagnosis, the inventor has found a group of gene sequence regions after extensive and in-depth research, which are CTSM-4F, CTSM-2BE, CTSM-3C and CTSM-4I. The methylation status of the sequence of these genes is significantly different between a tumor tissue and a non-tumor tissue. If abnormal methylation (hypermethylation) is detected in the promoter region of one of the above genes, the subject can be identified as having a high-risk of tumor.

Therefore, the disclosure provides an isolated polynucleotide of the human genome, comprising the nucleotide sequence shown in the sequence of SEQ ID NO: 1, 3, 5, or 7. For tumor cells from the patient, the polynucleotide contains 5-methylcytosine (5mC) at C positions of many 5'-CpG-3'. The disclosure also comprises a fragment of the polynucleotide of the sequence shown in SEQ ID NO: 1, 3, 5 or 7, having at least one (such as 2-40, more specifically 3, 5, 8, 10, 15, 20, 25, 30, 35) methylated CpG site. The above polynucleotides or fragments or complementary chains (antisense chains) can also be used in the design of detection agents or detection kits.

The above polynucleotides can be used as the key regions for analysis of the methylation status in the genome. Their methylation status can be analyzed by various technologies known in the art. Any technique that can be used to analyze the methylation state can be applied to the present disclosure.

When treated with bisulfite, un-methylated cytosine(s) of the above polynucleotides will be converted into uracil, while methylated cytosine(s) remained unchanged.

Therefore, the disclosure also provides the polynucleotides obtained from the above polynucleotides (including the complementary chain (antisense chain)) after being treated with bisulfite, including the polynucleotides of the sequence shown in SEQ ID NO: 2, 4, 6, or 8. These polynucleotides can also be used in the design of detection agents or detection kits.

The disclosure also comprises a fragment of the polynucleotides obtained from the above polynucleotides (including their complementary chains (antisense chains)) or the antisense chain thereof after being treated with bisulfite, wherein the fragment contains at least one methylated CpG site.

### Detection agents and kits

Based on the new discovery of the disclosure, a detection agent designed based on said polynucleotide(s) is also provided for detecting the methylation profile of polynucleotide(s) in the sample *in vitro.* The detection methods and agents known in the art for determining the sequence and methylation of the genome can be applied in the disclosure.

Therefore, the disclosure provides a method of preparing a tumor detection agent, including: providing the polynucleotide(s) (1, 2, 3 or 4 of the polynucleotides), designing a detection agent for specifically detecting a target sequence which is the full length or fragment of the polynucleotide; wherein, the target sequence has at least one methylated CpG site.

In a preferable embodiment, the target sequence comprises the nucleotide fragment of residues 240-296 of SEQ ID NO: 1; the nucleotide fragment of residues 279-323 of SEQ ID NO: 3; the nucleotide fragment of residues 186-235 of SEQ ID NO: 5; preferably, the primers are as shown in SEQ ID NO: 12, 16-18; or comprises the nucleotide fragment of residues 164-198 of SEQ ID NO: 7.

The detection agent includes but is not limited to: a primer, a probe, etc.

For example, the agent is a primer pair. Based on the sequence of the polynucleotide, those skilled in the art know how to design primer(s). The two primers are on each side of the specific sequence of the target gene to be amplified (including CpG sequence, for the gene region originally methylated, the primer is complementary with CpG, and for the gene region originally un-methylated, the primer is complementary with TpG).

The agent can also be a combination of agents (primer combination), including more than one set of primers, so that the multiple polynucleotides can be amplified respectively.

In a preferred embodiment of the disclosure, the primers are as shown in SEQ ID NO: 9-12; or as shown in SEQ ID NO: 12-15; or as shown in SEQ ID NO: 12, 16-18; or as shown in SEQ ID NO: 12, 19-21. These primers are used for nested PCR, and the amplified products after two runs of PCR are used for sequence identification. The products amplified by the above primers have suitable length and high specificity, even for the amplification of a complex system. These primers are especially suitable for methylation-specific PCR.

The disclosure also provides a kit for detecting the methylation profile of polynucleotide in a sample *in vitro,* which comprises container(s) and the above primer pair(s) in the container(s).

In addition, the kit can also include various reagents required for DNA extraction, DNA purification, PCR amplification, etc.

In addition, the kit can also include an instruction for use, which indicates operation steps of the detection and a result judgment standard, for the application of those skilled in the art.

### Detection method

The methylation profile of a polynucleotide can be determined by any technique in the art (such as methylation specific PCR (MSP) or real-time quantitative methylation specific PCR, Methylight), or other techniques that are still developing and will be developed.

Quantitative methylation specific PCR (QMSP) can also be used to detect methylation level. It is a continuous optical monitoring method based on fluorescent PCR, which is more sensitive than MSP. It has high throughput and avoids electrophoresis based result analysis.

Other available technologies include conventional methods in the art such as pyrosequencing, bisulfite converstion sequencing, qPCR, second generation sequencing, whole genome methylation sequencing, DNA enrichment detection, simplified bisulfite sequencing or HPLC, and combined gene group detection. It should be understood that, on the basis of the new disclosure herein, these well-known technologies and some technologies to be developed in the art can be applied to the present disclosure.

As a preferable embodiment of the disclosure, a method of detecting the methylation profile of a polynucleotide in a sample *in vitro* is also provided. The method is based on the follow principle: the un-methylated cytosine can be converted into uracil by bisulfite, which can be transformed into thymine in the subsequent PCR amplification process, while the methylated cytosine remains unchanged; therefore, after the polynucleotide is treated by bisulfite, the methylated site presents a polynucleotide polymorphism (SNP) similar to C/T. Based on the above principle, methylated and un-methylated cytosine can be distinguished by identifying the methylation profile of a polynucleotide in the sample.

The method of the disclosure includes: (a) providing samples and extracting genomic DNA; (b) treating the genomic DNA of step (a) with bisulfite, so as to convert the un-methylated cytosine in the genomic DNA into uracil; (c) analyzing whether the genomic DNA treated in step (b) contains an abnormal methylation profile.

The method of the disclosure can be used for: (i) analyzing whether a subject suffering from tumor by detecting the sample of the subject; (ii) identifying a high-risk population for tumor. The method may also be a case in which the purpose is not to obtain a direct disease diagnosis results.

In a preferable embodiment of the disclosure, DNA methylation is detected by PCR amplification and pyrosequencing. It should be understood by those in the art that DNA methylation detection is not limited to these methods, and any other DNA methylation detection method can also be used. The primers used in PCR amplification are not limited to those provided in Examples.

In a preferable embodiment of the disclosure, because of bisulfite treatment, in which un-methylated cytosine in genomic DNA are converted into uracil and then transformed into thymine in the subsequent PCR process, the sequence complexity of the genome will be reduced, and it will be more difficult to amplify specific target fragments by PCR. The inventor has found that, nested PCR can improve amplification efficiency and specificity, wherein two sets of primers (outer primers and inner primers) are used in two successive runs of PCR, and the product from the first run undergoes a second run with the second set of primers. It should be understood that the detection methods suitable for the present disclosure are not limited thereto.

After the research and verification on clinical samples, the method of the disclosure provides very high accuracy in the clinical diagnosis of tumors, therefore has a high clinical value.

The disclosure is further illustrated by the specific examples described below. It should be understood that these examples are merely illustrative, and do not limit the scope of the present disclosure. The experimental methods without specifying the specific conditions in the following examples generally used the conventional conditions, such as those described in J. Sambrook, Molecular Cloning: A Laboratory Manual (3rd ed. Science Press, 2002) or followed the manufacturer's recommendation.

### Example 1. Tumor marker CTSM-4F

### I. Description of the sequence for detection

In this example, the sequence of CTSM-4F tumor marker is shown in the SEQ ID NO: 1, in which the bases in each box are the potential methylated CpG site(s), and the optimal detection region is underlined.

SEQ ID NO: 1 are as follows:

CGCCCAGTAAGTTACGGAAAAGGCGGAGACAGAGGTTTCGTTCCCGCCCC
TCCAATTCAGTCTCCAAAAAGGTCCGCATAATTGATATATAAGGGGCTTCAGTGT
GTAGCAAAGTTGCAAAAGTTAAGAGTTGTTGTTTGTCTTCGATCATGTCTGGTAG
AGGCAAAGGTGGTAAAGGTTTAGGAAAGGGAGGCGCCAAGCGCCATCGCAAAG
TGCTGCGTGACAACATACAGGGCATCACGAAGCCCGCCATCCGTCGCTTGGCCC
GACGCGGCGGCGTGAAACGCATTTCGGGCCTCATTTATGAGGAGACCCGCGGT
GTTCTTAAGGTGTTCCTGGAGAATGTGATACGGGACGCCGTAACCTACACGGAG
CACGCCAAGCGTAAGACAGTCACTGCAATGGATGTTGTCTACGCGCTCAAGCGC
CAGGGACGCACTCTGTACGGCTTTGGTGGCTGAGCCTCACCCCGGCTTTTTATTT
AACAGCTCACCCATAAAAGGCCCTTTTCAGGGCCACCTCCTTCGTCACACGAAG
GGCTGTAACTGATGACGACTTGGGTTTCGTTTTGTAAATTTGGGATTCTAACTGA
GTTAAACCGAGCCGTTTTTAGCGATCTTCCTAAGATGGCGGATGTGCTAAGGAG
AAAGGGAAGGCGAAACATTAGAAACTTGTTCAGGTATTTCGATCGCAAACATT

### II. Detection step

1. Obtaining samples: selecting clinical tissue samples, including cancer tissue and corresponding paracarcinoma control tissue.
2. DNA extraction: extracting the genomic DNA of the sample by conventional phenol-chloroform method.
3. DNA methylation was detected by pyrosequencing as follows:
   (1) Bisulfite treatment: 5-200ng above extracted genomic DNA was treated with bisulfite using Methylation-Gold^{™} Kit (ZYMO Research, Cat. No.D5006) according to its instruction, and finally eluted with 20ul eluent and used as the template for subsequent PCR amplification.
   (2) SEQ ID NO: 2 was obtained after bisulfite treatment, wherein Y represents C (cytosine) or T (thymine). As compared with SEQ ID NO: 1, the original methylated cytosine C remains unchanged, but the original unmethylated cytosine is converted to T (U), therefor Y is used to represent C or T.

SEQ ID NO: 2 are as follows:

YGTTTAGTAAGTTAYGGAAAAGGYGGAGATAGAGGTTTYGTTTTYGTTTTT
TTAATTTAGTTTTTAAAAAGGTTYGTATAATTGATATATAAGGGGTTTTAGTGTG
TAGTAAAGTTGTAAAAGTTAAGAGTTGTTGTTTGTTTTYGATTATGTTTGGTAGA
GGTAAAGGTGGTAAAGGTTTAGGAAAGGGAGGYGTTAAGYGTTATYGTAAAG
TGTTGYGTGATAATATATAGGGTATTAYGAAGTTYGTTATTYGTYGTTTGGTTY
GAYGYGGYGGYGTGAAAYGTATTTYGGGTTTTATTTATGAGGAGATTYGYGGT
GTTTTTAAGGTGTTTTTGGAGAATGTGATAYGGGAYGTYGTAATTTATAYGGAG
TAYGTTAAGYGTAAGATAGTTATTGTAATGGATGTTGTTTAYGYGTTTAAGYGT
TAGGGAYGTATTTTGTAYGGTTTTGGTGGTTGAGTTTTATTTYGGTTTTTTATTTA
ATAGTTTATTTATAAAAGGTTTTTTTTAGGGTTATTTTTTTYGTTATAYGAAGGGT
TGTAATTGATGAYGATTTGGGTTTYGTTTTGTAAATTTGGGATTTTAATTGAGTT
AAATYGAGTYGTTTTTAGYGATTTTTTTAAGATGGYGGATGTGTTAAGGAGAAA
GGGAAGGYGAAATATTAGAAATTTGTTTAGGTATTTYGATYGTAAATATT

Based on SEQ ID NO: 2 and the characteristics of nested PCR and pyrosequencing, sets of amplification primers and sequencing primers were designed, as shown in Table 1 (corresponding to the sequence shown underlined or in bold in the SEQ ID NO: 2):

**Table 1**

| | Primer Name | Primer sequence |
|---|---|---|
| First run PCR | CTSM-4F-F1 | GTAGAGGTAAAGGTGGTAAAGGTTTAG(SEQ ID NO: 9) |
| | CTSM-4F-R1 | aaccttcaacaccccaaccatataAAAACACCRCRAATC TCCTCATAAAT(SEQ ID NO: 10) |
| Second run PCR | CTSM-4F-F2 | TGTTGYGTGATAATATATAGGGTATT(SEQ ID NO: 11) |
| | CTSM-4F-R2 (biotin modified at 5' terminal) | aaccttcaacaccccaaccatata(SEQ ID NO: 12) (corresponding to the lowercase and italicized part of CTSM-4F-R1) |

(3) Nested PCR: using 2 × PCR Master Mix (Lifefeng Biotech Co., Ltd, Cat No. PT102), but not limited thereto.

Amplification system of the first run of PCR was as follows:

| Component | Volume |
|---|---|
| 2×PCR Mix | 5ul |
| **CTSM-4F-F1(10uM)** | 0.1 ul |
| **CTSM-4F-R1(10uM)** | 0.1 ul |
| Template | 1ul |
| ddH₂O | 3.8ul |

Amplification procedure of the first run of PCR was as follows:

| | | |
|---|---|---|
| Pre-denaturation | 98°C | 30s |
| Amplification of 30 cycles | 98 °C | 10s |
| | 58°C | 30s |
| | 72°C | 30s |
| Extension | 72°C | 3min |
| Preservation | 4°C | |

After the first run of PCR, its products were used as templates for the second run of PCR. The amplification system of the second run of PCR was as follows:

| | |
|---|---|
| 2×PCR Mix | 13ul |
| CTSM-4F-F2 **(10uM)** | 0.5ul |
| CTSM-4F-R2 (biotin modified at 5' terminal) **(10uM)** | 0.5ul |
| Template | 4ul |
| ddH₂O | 12ul |

Amplification procedure of the second run of PCR was as follows:

| | | |
|---|---|---|
| Pre-denaturation | 98°C | 30s |
| Amplification of 30 cycles | 98°C | 10s |
| | 58°C | 30s |
| | 72°C | 30s |
| Extension | 72°C | 3min |
| Preservation | 4°C | |

(4) Agarose gel electrophoresis: to identify the specificity of PCR products, the length of the amplified product was detected by agarose gel electrophoresis, which should be 177bp.

(5) Pyrosequencing: the methylation value of each CpG site in the target region was detected through the pyrosequencing instrument (QIAGEN) according to the steps in the instruction.

(6) Analysis and calculation: the methylation values of 11 CpG sites were detected by pyrosequencing, and their average value was calculated as the final methylation value of the sample in this region. Although the average value is used here, other calculation such as sum or median of the methylation values can be used.

### III. Result

By analyzing the methylation values of samples, the tumor samples can be effectively distinguished from the control samples. CTSM-4F is hypomethylated in the control samples, while in the tumor samples, CTSM-4F is hypermethylated.

### IV. Clinical detection and analysis

### 1. Detection and analysis of rectal cancer

Among the confirmed rectal cancer samples obtained from the hospital, 4 cases of colorectal cancer samples were randomly selected, and the corresponding paracarcinoma tissues were used as control samples.

Using the above methods of II to IV, the DNA from these samples was obtained and subjected to bisulfite treatment, nested PCR, agarose gel electrophoresis, pyrosequencing, and analysis and calculation of the methylation values of CpG sites.

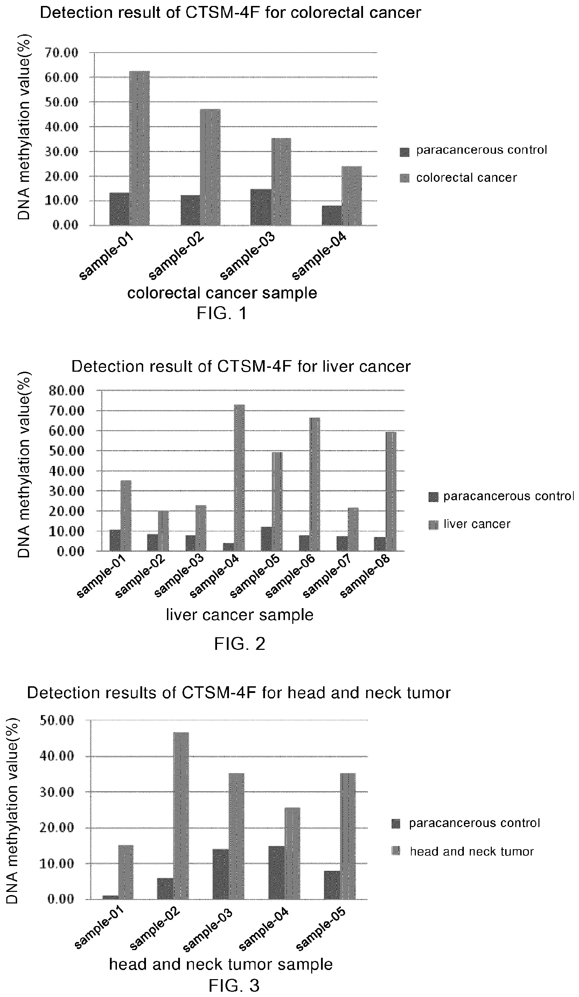

As shown in Figure 1, results indicated that the methylation level of CTSM-4F in control samples was significantly lower than that in colorectal cancer samples, and CTSM-4F was significantly abnormal hypermethylated in colorectal cancer, which can be used as an excellent marker for colorectal cancer. Further expansion of the clinical samples showed the same significance.

### 2. Detection and analysis of liver cancer

Among the confirmed liver cancer samples obtained from the hospital, 8 cases of liver cancer samples were randomly selected, and the corresponding paracarcinoma tissues were used as control samples.

Using the above methods of II to IV, DNA was obtained from these samples and subjected to bisulfite treatment, nested PCR, agarose gel electrophoresis, pyrosequencing, and analysis and calculation of the methylation value of CpG sites.

As shown in Figure 2, results indicated that the methylation level of CTSM-4F in control samples was significantly lower than that in liver cancer samples, and CTSM-4F was significantly abnormal hypermethylated in liver cancer, which can be used as an excellent marker for liver cancer. Further expansion of the clinical samples showed the same significance.

### 3. Detection and analysis of head and neck tumor

Among the confirmed head and neck tumor samples obtained from the hospital, 5 cases of head and neck tumor samples were randomly selected, and the corresponding paracarcinoma tissues were used as control samples.

Using the above methods of II to IV, DNA was obtained from these samples and subjected to bisulfite treatment, nested PCR, agarose gel electrophoresis, pyrosequencing, and analysis and calculation of the methylation value of CpG sites.

As shown in Figure 3, results indicated that the methylation level of CTSM-4F in control samples was significantly lower than that in head and neck tumor samples, and CTSM-4F was significantly abnormal hypermethylated in head and neck tumor, which can be used as an excellent marker for head and neck tumor. Further expansion of the clinical samples showed the same significance.

### 4. Detection and analysis of lung cancer

Among the confirmed lung cancer samples obtained from the hospital, 5 cases of lung cancer plasma samples were randomly selected as the experimental group, and 5 cases of normal plasma samples were selected as the control group.

Using the above methods of II to IV, DNA was obtained from these samples and subjected to bisulfite treatment, nested PCR, agarose gel electrophoresis, pyrosequencing, and analysis and calculation of the methylation value of CpG sites.

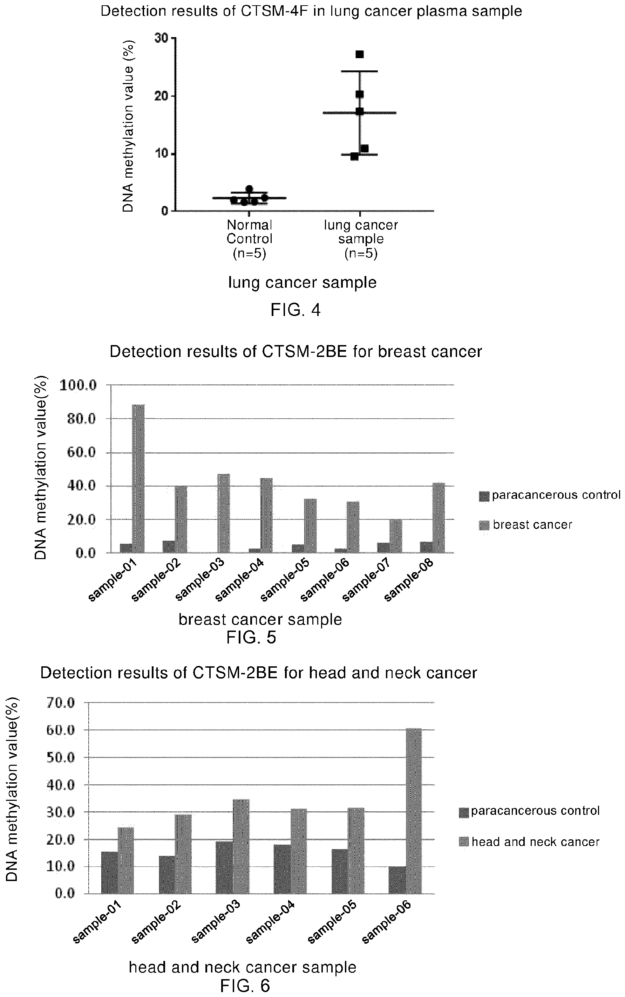

As shown in Figure 4, results indicated that the methylation level of CTSM-4F of the control group was significantly lower than that of the experimental group, and CTSM-4F was significantly abnormal hypermethylated in lung cancer, which can be used as an excellent marker for lung cancer. Further expansion of the clinical samples showed the same significance.

### Example 2. Tumor marker CTSM-2BE

### I. Description of the sequence for detection

In this example, the sequence of CTSM-2BE tumor marker is shown in the SEQ ID NO: 3, in which the bases in each box are the potential methylated CpG site(s). The optimal detection region is underlined.

SEQ ID NO: 3 are as follows:
AGCTGTTTGTTAAATAGGCTTTCATTTTAATTTTTTAAAAAATATTTTCACTAGTT
AGACGGAATATTTATTTCTGATCCTCGTTGTAGGGCTAAACTAGGTGTGACTTGC
TTTTCCATTAAGGACTGTAGCCATTTTGGCTAAGAAAGTCTTTCCTGTCTTAGCTC
TCTTCAAGAGATGCTATTTACTTTTTTGTTGTTGTTTTCTCCGGAGCTTTATTGCCT
ACCCTTTATTTTCACGATGTGTTTGTGCATTCTGTAAAAAGTGGGAAACACTGTT
CTCCGGATTGTGTGGGTGATCGCCGGTCGCCTAGCTTGCCGTTCTTCAGGTATCT
CCTGCTGCTCCAGAATCTACATCTGAATGTCAAGGCATAGCTTTTCCAGGAGCTT
TTAAACCGACCTTTCAATTCGAAGACGTAACTGCGCCAGGAGCTTTGTCTCCCTG
GCATATAAAAGCATAGAAGAGAGCAACTTCTGGTTTTTAAAATAAGTAAACTAA
TCTGAATTGTTTGCAATGGTAGGAACTTGTTATATAAAATGTTAATTAGGTGGCC
CGTGCTCGAAAACTGCTCTCAGGATATGACCAATGGGAGAGTAGACCTAAGCTC
CTTCATTTGCATGCAGA

SEQ ID NO: 4 was obtained after bisulfite treatment, wherein Y represents C (cytosine) or T (thymine).

SEQ ID NO: 4 are as follows:
AGTTGTTTGTTAAATAGGTTTTTATTTTAATTTTTTAAAAAATATTTTTATTA
GTTAGAYGGAATATTTATTTTTGATTTTYGTTGTAGGGTTAAATTAGGTGTGATT
TGTTTTTTTATTAAGGATTGTAGTTATTTTGGTTAAGAAAGTTTTTTTTGTTTTAGT
TTTTTTTAAGAGATGTTATTTATTTTTTTGTTGTTGTTTTTTTYGGAGTTTTATTGT
TTATTTTTTATTTTTAYGATGTGTTTGTGTATTTTGTAAAAAGTGGGAAATATT
GTTTTTYGGATTGTGTGGGTGATYGTYGGTYGTTTAGTTTGTYGTTTTTTAGGT
ATTTTTTGTTGTTTTAGAATTTATATTTGAATGTTAAGGTATAGTTTTTTTAGGAG
TTTTTAAATYGATTTTTTAATTYGAAGAYGTAATTGYGTTAGGAGTTTTGTTTTTT
TGGTATATAAAAGTATAGAAGAGAGTAATTTTTGGTTTTTAAAATAAGTAAATTA
ATTTGAATTGTTTGTAATGGTAGGAATTTGTTATATAAAATGTTAATTAGGTGGTT
YGTGTTYGAAAATTGTTTTTAGGATATGATTAATGGGAGAGTAGATTTAAGTTTT
TTTATTTGTATGTAGA

Based on SEQ ID NO: 4 and the characteristics of nested PCR and pyrosequencing, sets of amplification primers and sequencing primers were designed and showed in Table 2.

**Table 2**

| | Primer Name | Primer sequence |
|---|---|---|
| First run | CTSM-2BE-F1 | GATGTGTTTGTGTATTTTGTAAAAAGTG(SEQ ID NO: 13) |
| PCR | CTSM-2BE-R1 | aaccttcaacaccccaaccatataTATACCTTAACATTCAAA TATAAATTC(SEQ ID NO: 14) |
| Second run PCR | CTSM-2BE-F2 | TTTTYGGATTGTGTGGGTGA(SEQ ID NO: 15) |
| | CTSM-2BE-R2 (biotin modified at 5' terminal) | aaccttcaacaccccaaccatata(SEQ ID NO: 12) |

By use of the same methods as Example 1 and the above primers, DNA was extracted from samples and subjected to bisulfite treatment, nested PCR, agarose gel electrophoresis, pyrosequencing, and analysis and calculation of the methylation value of CpG sites.

### II. Clinical detection and analysis

### 1. Detection and analysis of breast cancer

Among the confirmed breast cancer samples obtained from the hospital, 8 cases of breast cancer samples were randomly selected, and the corresponding paracarcinoma tissues were used as control samples.

DNA was obtained from these samples and subjected to bisulfite treatment, nested PCR, agarose gel electrophoresis, pyrosequencing, and analysis and calculation of the methylation value of CpG sites.

As shown in Figure 5, results indicated that the methylation level of CTSM-2BE in control samples was significantly lower than that in breast cancer samples, and CTSM-2BE was significantly abnormal hypermethylated in breast cancer, which can be used as an excellent marker for breast cancer. Further expansion of the clinical samples showed the same significance.

### 2. Detection and analysis of head and neck tumor

Among the confirmed head and neck tumor samples obtained from the hospital, 6 cases of head and neck tumor samples were randomly selected, and the corresponding paracarcinoma tissues were used as control samples.

DNA was obtained from these samples and subjected to bisulfite treatment, nested PCR, agarose gel electrophoresis, pyrosequencing, and analysis and calculation of the methylation value of CpG sites.

As shown in Figure 6, results indicated that the methylation level of CTSM-2BE in control samples was significantly lower than that in head and neck tumor samples, and CTSM-2BE was significantly abnormal hypermethylated in head and neck tumor, which can be used as an excellent marker for head and neck tumor. Further expansion of the clinical samples showed the same significance.

### 3. Detection and analysis of lung cancer

Among the confirmed lung cancer samples obtained from the hospital, 8 cases of lung cancer samples were randomly selected, and the corresponding paracarcinoma tissues were used as control samples.

DNA was obtained from these samples and subjected to bisulfite treatment, nested PCR, agarose gel electrophoresis, pyrosequencing, and analysis and calculation of the methylation value of CpG sites.

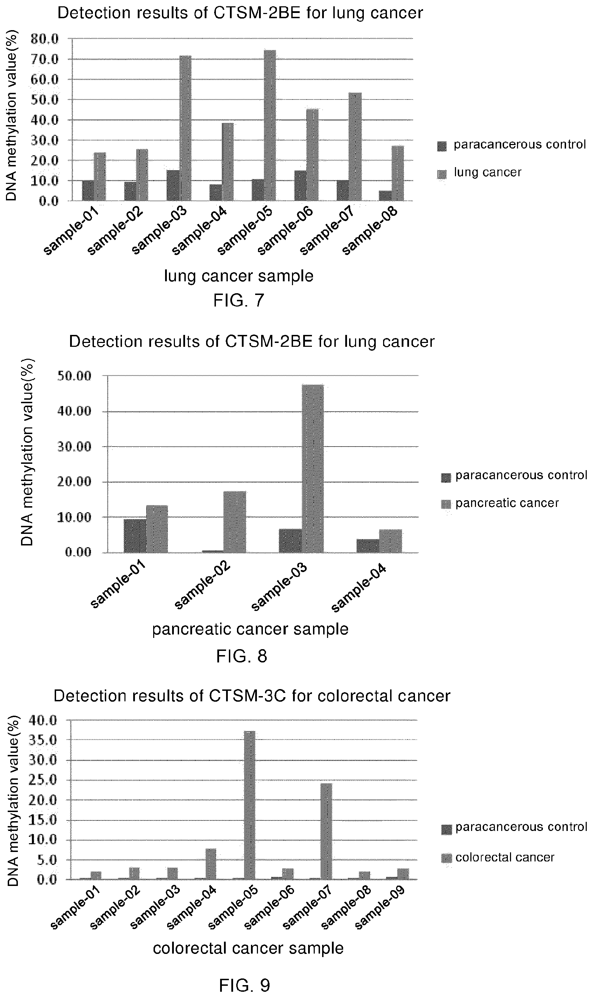

As shown in Figure 7, results indicated that the methylation level of CTSM-2BE in control samples was significantly lower than that in lung cancer samples, and CTSM-2BE was significantly abnormal hypermethylated in lung cancer, which can be used as an excellent marker for lung cancer. Further expansion of the clinical samples showed the same significance.

### 4. Detection and analysis of pancreatic cancer

Among the confirmed pancreatic cancer samples obtained from the hospital, 8 cases of pancreatic cancer samples were randomly selected, and the corresponding paracarcinoma tissues were used as control samples.

DNA was obtained from these samples and subjected to bisulfite treatment, nested PCR, agarose gel electrophoresis, pyrosequencing, and analysis and calculation of the methylation value of CpG sites.

As shown in Figure 8, results indicated that the methylation level of CTSM-2BE in control samples was significantly lower than that in pancreatic cancer samples, and CTSM-2BE was significantly abnormal hypermethylated in pancreatic cancer, which can be used as an excellent marker for pancreatic cancer. Further expansion of the clinical samples showed the same significance.

### Example 3. Tumor marker CTSM-3C

### I. Description of the Detection Sequence

In this example, the sequence of CTSM-3C tumor marker is shown in the SEQ ID NO: 5, in which the bases in each box are the potential methylated CpG site(s). The optimal detection region is underlined.

SEQ ID NO: 5 are as follows:

TGGGGCAACTCATCCAATAAGATTGTCTAGTAATGAACCAATCAGTCTGGTCACT
CTTCAGCCAATGATTTTATCGCGCGGGACTTTTGAAATATTACAGGACCAATCA
GAATGTTTCTCACTATATTTAAAGGCCACTTGCTCTCAGTTCACTACACTTTTGTG
TGTGCTCTCATTGCAAATGGCTCGTACGAAGCAAACAGCTCGCAAGTCTACCG
GCGGCAAAGCTCCGCGCAAGCAGCTTGCTACTAAAGCAGCCCGTAAGAGCG
CTCCGGCCACCGGTGGCGTGAAGAAACCTCATCGCTACCGCCCGGGCACC
GTGGCCTTGCGCGAAATCCGTCGCTACCAGAAGTCCACCGAGCTGCTGATC
CGGAAGCTGCCGTTCCAGCGCCTGGTGCGAGAAATCGCCCAGGACTTCAAA
ACCGACCTGCGTTTCCAGAGCTCTGCGGTGATGGCGCTGCAGGAGGCTTGT
GAGGCCTACCTGGTGGGACTCTTCGAAGACACCAATCTGTGCGCTATTCAC
GCTAAACGCGTCACCATCATGCCCAAAGATATCCAGCTGGCACGTCGCATC
CGTGGGGAAAGGGCATAAGTCTGCCCGTTTCTTCCTCATTGAAAAGGCTCTTT
TCAGAGCCACTCACAATTTCACTTAAAAACAGTTGTAACCCA

SEQ ID NO: 6 was obtained after bisulfite treatment, wherein Y represents C (cytosine) or T (thymine):
SEQ ID NO: 6 are as follows:

TGGGGTAATTTATTTAATAAGATTGTTTAGTAATGAATTAATTAGTTTGGTTATTT
TTTAGTTAATGATTTTATYGYGYGGGATTTTTGAAATATTATAGGATTAATTAGA
ATGTTTTTTATTATATTTAAAGGTTATTTGTTTTTAGTTTATTATATTTTTGTGTGT
GTTTTTATTGTAAATGGTTYGTAYGAAGTAAATAGTTYGTAAGTTTATYGGYG
GTAAAGTTTYGYGTAAGTAGTTTGTTATTAAAGTAGTTYGTAAGAGYGTTTY
GGTTATYGGTGGYGTGAAGAAATTTTATYGTTATYGTTYGGGTATYGTGGT
TTTGYGYGAAATTYGTYGTTATTAGAAGTTTATYGAGTTGTTGATTYGGAAG
TTGTYGTTTTAGYGTTTGGTGYGAGAAATYGTTTAGGATTTTAAAATYGATT
TGYGTTTTTAGAGTTTTGYGGTGATGGYGTTGTAGGAGGTTTGTGAGGTTT
ATTTGGTGGGATTTTTYGAAGATATTAATTTGTGYGTTATTTAYGTTAAAYG
YGTTATTATTATGTTTAAAGATATTTAGTTGGTAYGTYGTATTYGTGGGGAA
AGGGTATAAGTTTGTTYGTTTTTTTTTTATTGAAAAGGTTTTTTTTAGAGTTATT
TATAATTTTATTTAAAAATAGTTGTAATTTA

Based on SEQ ID NO: 6 and the characteristics of nested PCR and pyrosequencing, sets of amplification primers and sequencing primers are designed and showed in Table 3.

**Table 3**

| | Primer Name | Primer sequence |
|---|---|---|
| First run | CTSM-3C-F1 | TTTGTTTTTAGTTTATTATATTTTT**GTGTG**(SEQ ID NO: 16) |
| PCR | CTSM-3C-R1 | aaccttcaacaccccaaccatataAACTACTTTAATAACAAA CTACTTAC(SEQ ID NO: 17) |
| Second run PCR | CTSM-3C-F2 | **GTGTGTGTTTTTATTGTAAATGG**(SEQ ID NO: 18) |
| | CTSM-3C-R2 (biotin modified at 5' terminal) | Aaccttcaacaccccaaccatata (SEQ ID NO: 12) |

By use of the same methods as Example 1 and the above primers, DNA was extracted from samples and subjected to bisulfite treatment, nested PCR, agarose gel electrophoresis, pyrosequencing, and analysis and calculation of the methylation value of CpG sites.

### II. Clinical detection and analysis

### 1. Detection and analysis of colorectal cancer

Among the confirmed colorectal cancer samples obtained from the hospital, 9 cases of colorectal cancer samples were randomly selected, and the corresponding paracarcinoma tissues were used as control samples.

DNA was obtained from these samples and subjected to bisulfite treatment, nested PCR, agarose gel electrophoresis, pyrosequencing, and analysis and calculation of the methylation value of CpG sites.

As shown in Figure 9, results indicated that the methylation level of CTSM-3C in control samples was significantly lower than that in colorectal cancer samples, and CTSM-3C was significantly abnormal hypermethylated in colorectal cancer, which can be used as an excellent marker for colorectal cancer. Further expansion of the clinical samples showed the same significance.

### 2. Detection and analysis of head and neck cancer

Among the confirmed head and neck cancer samples obtained from the hospital, 5 cases of head and neck cancer samples were randomly selected, and the corresponding paracarcinoma tissues were used as control samples.

DNA was obtained from these samples and subjected to bisulfite treatment, nested PCR, agarose gel electrophoresis, pyrosequencing, and analysis and calculation of the methylation value of CpG sites.

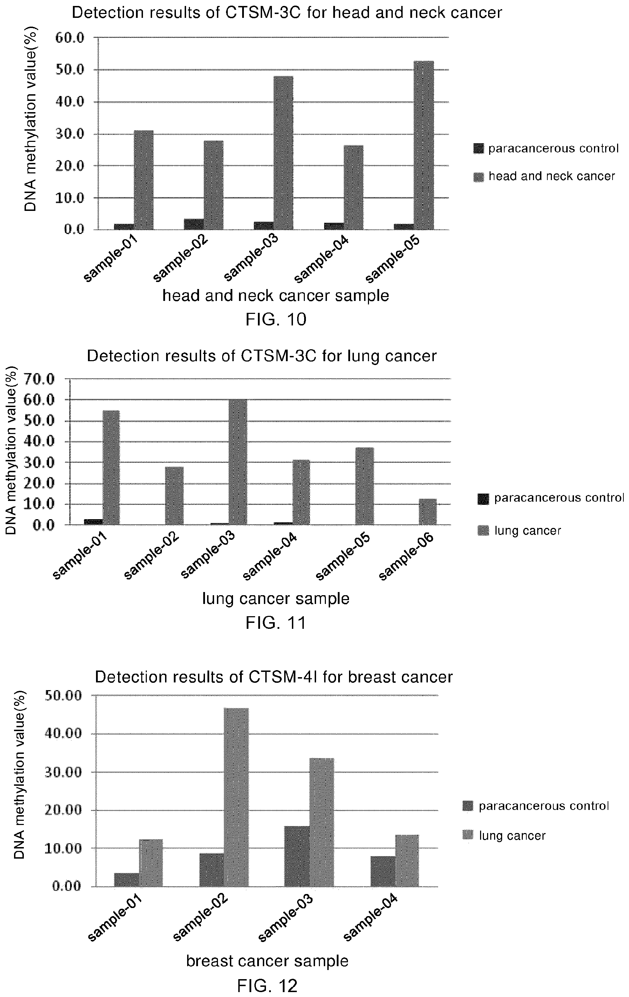

As shown in Figure 10, results indicated that the methylation level of CTSM-3C in control samples was significantly lower than that in head and neck cancer samples, and CTSM-3C was significantly abnormal hypermethylated in head and neck cancer, which can be used as an excellent marker for head and neck cancer. Further expansion of the clinical samples showed the same significance.

### 3. Detection and analysis of lung cancer

Among the confirmed lung cancer samples obtained from the hospital, 6 cases of lung cancer samples were randomly selected, and the corresponding paracarcinoma tissues were used as control samples.

DNA was obtained from these samples and subjected to bisulfite treatment, nested PCR, agarose gel electrophoresis, pyrosequencing, and analysis and calculation of the methylation value of CpG sites.

As shown in Figure 11, results indicated that the methylation level of CTSM-3C in control samples was significantly lower than that in lung cancer samples, and CTSM-3C was significantly abnormal hypermethylated in lung cancer, which can be used as an excellent marker for lung cancer. Further expansion of the clinical samples showed the same significance.

### Example 4. Tumor marker CTSM-4I

### I. Description of the sequence for detection

In this example, the sequence of CTSM-4I tumor marker is shown in the SEQ ID NO: 7, in which the bases in each box are the potential methylated CpG site(s). The optimal detection region is underlined.

SEQ ID NO: 7 are as follows:

TACCCATAAAAGAAAGCTGCCATCACAGGCAGCAGACCTTTGTTCTCTGACCACT
TGATAATGTCAGGACGCGGCAAAGGAGGTAAGGGCCTGGGGAAAGGGGGTGCC
AAGCGCCACCGCAAGGTGCTGCGCGACAACATCCAGGGTATCACCAAGCCAGCC
ATTCGGCGCCTTGCTCGCCGCGGCGGCGTGAAGCGCATTTCTGGCCTCATCT
ATGAGGAGACCCGCGGAGTGTTGAAGGTGTTCCTGGAGAACGTGATCCGGGAC
GCCGTGACCTACACGGAGCACGCCAAGCGCAAGACGGTCACCGCCATGGACGT
GGTCTACGCGCTCAAGCGCCAGGGCCGCACCCTCTATGGCTTCGGCGGCTAAAT

SEQ ID NO: 8 was obtained after bisulfite treatment, wherein Y represents C (cytosine) or T (thymine):
SEQ ID NO: 8 are as follows:

TATTTATAAAAGAAAGTTGTTATTATAGGTAGTAGATTTTTGTTTTTTGATTATTT
GATAATGTTAGGAYGYGGTAAAGGAGGTAAGGGTTTGGGGAAAGGGGGTGTTA
AGYGTTATYGTAAGGTGTTGYGYGATAATATTTAGGGTATTATTAAGTTAGTT
ATTYGGYGTTTTGTTYGTYGYGGYGGYGTGAAGYGTATTTTTGGTTTTATTTA
TGAGGAGATTYGYGGAGTGTTGAAGGTGTTTTTGGAGAAYGTGATTYGGGAYG
TYGTGATTTATAYGGAGTAYGTTAAGYGTAAGAYGGTTATYGTTATGGAYGTG
GTTTAYGYGTTTAAGYGTTAGGGTYGTATTTTTTATGGTTTYGGYGGTTAAAT

Based on SEQ ID NO: 8 and the characteristics of nested PCR and pyrosequencing, sets of amplification primers and sequencing primers are designed and showed in Table 4.

**Table 4**

| | Primer Name | Primer sequence |
|---|---|---|
| First run PCR | CTSM-4I-F1 | GGGTGTTAAGYGTTATYGTAAGGTGTTG(SEQ ID NO: 19) |
| | CTSM-4I-R1 | aaccttcaacaccccaaccatataCTCCTCATAAATAAAACCA AAAATAC(SEQ ID NO: 20) |
| First run PCR | CTSM-4I-F2 | **TTTAGGGTATTATTAAGTTAGTTA**(SEQ ID NO: 21) |
| | CTSM-4I-R2 (biotin modified at 5' terminal) | aaccttcaacaccccaaccatata(SEQ ID NO: 12) |

### II. Clinical detection and analysis

### 1. Detection and analysis of breast cancer

Among the confirmed breast cancer samples obtained from the hospital, 4 cases of breast cancer samples were randomly selected, and the corresponding paracarcinoma tissues were used as control samples.

DNA was obtained from these samples and subjected to bisulfite treatment, nested PCR, agarose gel electrophoresis, pyrosequencing, and analysis and calculation of the methylation value of CpG sites.

As shown in Figure 12, results indicated that the methylation level of CTSM-4I in control samples was significantly lower than that in breast cancer samples, and CTSM-4I was significantly abnormal hypermethylated in breast cancer, which can be used as an excellent marker for breast cancer.

### 2. Detection and analysis of colorectal cancer

Among the confirmed colorectal cancer samples obtained from the hospital, 5 cases of colorectal cancer samples were randomly selected, and the corresponding paracarcinoma tissues were used as control samples.

DNA was obtained from these samples and subjected to bisulfite treatment, nested PCR, agarose gel electrophoresis, pyrosequencing, and analysis and calculation of the methylation value of CpG sites.

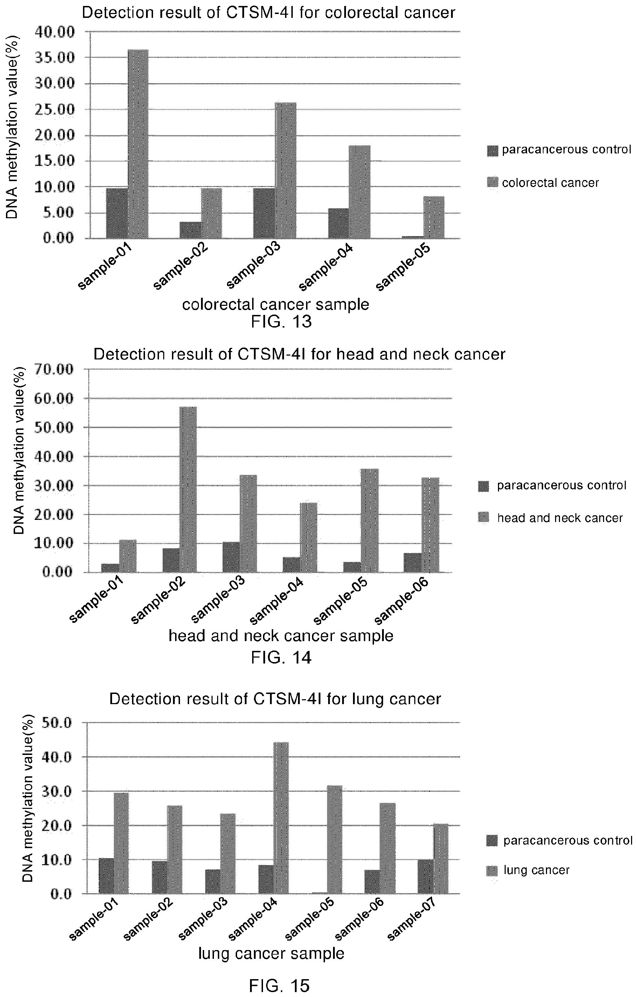

As shown in Figure 13, results indicated that the methylation level of CTSM-4I in control samples was significantly lower than that in colorectal cancer samples, and CTSM-4I was significantly abnormal hypermethylated in colorectal cancer, which can be used as an excellent marker for colorectal cancer.

### 3. Detection and analysis of head and neck cancer

Among the confirmed head and neck cancer samples obtained from the hospital, 6 cases of head and neck cancer samples were randomly selected, and the corresponding paracarcinoma tissues were used as control samples.

DNA was obtained from these samples and subjected to bisulfite treatment, nested PCR, agarose gel electrophoresis, pyrosequencing, and analysis and calculation of the methylation value of CpG sites.

As shown in Figure 14, results indicated that the methylation level of CTSM-4I in control samples was significantly lower than that in head and neck cancer samples, and CTSM-4I was significantly abnormal hypermethylated in head and neck cancer, which can be used as an excellent marker for head and neck cancer. Further expansion of the clinical samples showed the same significance.

### 4. Detection and analysis of lung cancer

Among the confirmed lung cancer samples obtained from the hospital, 7 cases of lung cancer samples were randomly selected, and the corresponding paracarcinoma tissues were used as control samples.

DNA was obtained from these samples and subjected to bisulfite treatment, nested PCR, agarose gel electrophoresis, pyrosequencing, and analysis and calculation of the methylation value of CpG sites.

As shown in Figure 15, results indicated that the methylation level of CTSM-4I in control samples was significantly lower than that in lung cancer samples, and CTSM-4I was significantly abnormal hypermethylated in lung cancer, which can be used as an excellent marker for lung cancer. Further expansion of the clinical samples showed the same significance.

## Claims

1. A method for cancer detection *in vitro,* comprising detecting the 5-methylation profile of (a) target sequence(s) comprising:
(a) the polynucleotide with the nucleotide sequence as shown in SEQ ID NO: 1, wherein the target sequence comprises at least the nucleotide fragment of residues 240-296 of SEQ ID NO: 1;
(b) the polynucleotide with the nucleotide sequence as shown in SEQ ID NO: 3, wherein the target sequence comprises at least the nucleotide fragment of residues 279-323 of SEQ ID NO: 3;
(c) the polynucleotide with the nucleotide sequence as shown in SEQ ID NO: 5, wherein the target sequence comprises at least the nucleotide fragment of residues 186-235 of SEQ ID NO: 5;
(d) the polynucleotide with the nucleotide sequence shown in SEQ ID NO: 7, wherein the target sequence comprises at least the nucleotide fragment of residues 164-198 of SEQ ID NO: 7;
and/or
(e) a nucleic acid complementary to the polynucleotide or fragment of (a)-(d);
wherein the cancer is selected from the group consisting of rectal cancer, liver cancer, head and neck cancer, lung cancer, breast cancer, pancreatic cancer, and colorectal cancer.

2. The method for cancer detection according to claim 1, wherein the fragment of the polynucleotide of (a)-(d) consists of nucleotides 240-296 of SEQ ID NO: 1, nucleotides 279-323 of SEQ ID NO: 3, nucleotides 186-235 of SEQ ID NO: 5, or nucleotide 164-198 of SEQ ID NO: 7, respectively.

3. Use of
(a) the polynucleotide with the nucleotide sequence as shown in SEQ ID NO: 1, wherein the target sequence comprises at least the nucleotide fragment of residues 240-296 of SEQ ID NO: 1;
(b) the polynucleotide with the nucleotide sequence as shown in SEQ ID NO: 3, wherein the target sequence comprises at least the nucleotide fragment of residues 279-323 of SEQ ID NO: 3;
(c) the polynucleotide with the nucleotide sequence as shown in SEQ ID NO: 5, wherein the target sequence comprises at least the nucleotide fragment of residues 186-235 of SEQ ID NO: 5;
(d) the polynucleotide with the nucleotide sequence as shown in SEQ ID NO: 7, wherein the target sequence comprises at least the nucleotide fragment of residues 164-198 of SEQ ID NO: 7;
and/or
(e) a nucleic acid complementary to the polynucleotide or fragment of (a)-(d), in the manufacturing of a cancer detection agent or kit, wherein the nucleic acid target sequence has at least one methylated CpG site; and
wherein the cancer is selected from the group consisting of rectal cancer, liver cancer, head and neck cancer, lung cancer, breast cancer, pancreatic cancer, and colorectal cancer.

4. A method of preparing a cancer detection agent, comprising: providing
(a) the polynucleotide with the nucleotide sequence as shown in SEQ ID NO: 1, wherein the target sequence comprises at least the nucleotide fragment of residues 240-296 of SEQ ID NO: 1;
(b) the polynucleotide with the nucleotide sequence as shown in SEQ ID NO: 3, wherein the target sequence comprises at least the nucleotide fragment of residues 279-323 of SEQ ID NO: 3;
(c) the polynucleotide with the nucleotide sequence as shown in SEQ ID NO: 5, wherein the target sequence comprises at least the nucleotide fragment of residues 186-235 of SEQ ID NO: 5;
(d) the polynucleotide with the nucleotide sequence as shown in SEQ ID NO: 7, wherein the target sequence comprises at least the nucleotide fragment of residues 164-198 of SEQ ID NO: 7;
and/or
(e) a nucleic acid complementary to the polynucleotide or fragment of (a)-(d)
, designing a detection agent for specifically detecting a target sequence which is the full length or fragment of the polynucleotide; wherein the nucleic acid target sequence has at least one methylated CpG site; preferably, the detection agent comprises: a primer, a probe.

5. An agent or a combination of agents, whereby said agent or combination of agents includes but is not limited to a primer,
wherein the agent or the combination of agents specifically detect the 5-methylation on CPG site(s) of a target sequence,
wherein, the target sequence is of the nucleotide sequence as shown in SEQ ID NO: 1, and the target sequence comprises the nucleotide fragment of residues 240-296 of SEQ ID NO: 1, and wherein the primer is as shown in SEQ ID NO: 9-12;
the target sequence is of the nucleotide sequence as shown in SEQ ID NO: 3, and the target sequence comprises the nucleotide fragment of residues 279-323 of SEQ ID NO:3, and wherein the primer is as shown in SEQ ID NO: 12-15;
the target sequence is of the nucleotide sequence as shown in SEQ ID NO: 5, and the target sequence comprises the nucleotide fragment of residues 186-235 of SEQ ID NO:5; and wherein the primer is as shown in SEQ ID NO: 12, 16-18, and/or
the target sequence is of the nucleotide sequence as shown in SEQ ID NO: 7, and the target sequence comprises the nucleotide fragment of residues 164-198 of SEQ ID NO:7, and wherein the primer is as shown in SEQ ID NO: 12, 19-21.

6. Use of the agent or combination of agents according to claim 5 in the manufacturing of a kit for detecting cancer, wherein the cancer is selected from the group consisting of rectal cancer, liver cancer, head and neck cancer, lung cancer, breast cancer, pancreatic cancer, and colorectal cancer.

7. A cancer detection kit, comprising:
container(s) and the agent or combination of agents according to claim 5 in the container(s), wherein the cancer is selected from the group consisting of rectal cancer, liver cancer, head and neck cancer, lung cancer, breast cancer, pancreatic cancer, and colorectal cancer.

8. A method of detecting the 5-methylation profile of
(a) the polynucleotide with the nucleotide sequence as shown in SEQ ID NO: 1, wherein the target sequence comprises at least the nucleotide fragment of residues 240-296 of SEQ ID NO: 1;
(b) the polynucleotide with the nucleotide sequence as shown in SEQ ID NO: 3, wherein the target sequence comprises at least the nucleotide fragment of residues 279-323 of SEQ ID NO: 3;
(c) the polynucleotide with the nucleotide sequence as shown in SEQ ID NO: 5, wherein the target sequence comprises at least the nucleotide fragment of residues 186-235 of SEQ ID NO: 5;
(d) the polynucleotide with the nucleotide sequence as shown in SEQ ID NO: 7, wherein the target sequence comprises at least the nucleotide fragment of residues 164-198 of SEQ ID NO: 7;
and/or
(e) a nucleic acid complementary to the polynucleotide or fragment of (a)-(d) in a sample *in vitro,* comprising:
(i) providing the sample and extracting DNA;
(ii) treating the sample to be detected to convert the unmodified cytosine into uracil, treating the DNA of step (i) with bisulfite;
(iii) analyzing the methylated CpG sites of said polynucleotide in the genomic DNA treated by step (ii).

9. The method according to claim 8, wherein, in step (3), the analysis methods include: pyrosequencing, bisulfite conversion sequencing, qPCR, second generation sequencing, whole genome methylation sequencing, DNA enrichment detection, simplified bisulfite sequencing technology, HPLC, or their combination.

## Patentansprüche

1. Verfahren zur Krebserkennung in Vitro, umfassend Erkennen des 5-Methylierungsprofils von (a) einer Zielsequenz/Zielsequenzen, umfassend
(a) das Polynucleotid mit der Nucleotidsequenz, wie in SEQ ID NO. 1 gezeigt, wobei die Zielsequenz mindestens das Nucleotidfragment der Reste 240-296 von SEQ ID NO. 1 umfasst;
(b) das Polynucleotid mit der Nucleotidsequenz, wie in SEQ ID NO. 3 gezeigt, wobei die Zielsequenz mindestens das Nucleotidfragment der Reste 279-323 von SEQ ID NO. 3 umfasst;
(c) das Polynucleotid mit der Nucleotidsequenz, wie in SEQ ID NO. 5 gezeigt, wobei die Zielsequenz mindestens das Nucleotidfragment der Reste 186-235 von SEQ ID NO. 5 umfasst;
(d) das Polynucleotid mit der Nucleotidsequenz, die in SEQ ID NO. 7 gezeigt ist, wobei die Zielsequenz mindestens das Nucleotidfragment der Reste 164-198 von SEQ ID NO. 7 umfasst;
und/oder
(e) eine Nucleinsäure, die zu dem Polynucleotid oder Fragment von (a)-(d) komplementär ist, wobei der Krebs aus der Gruppe ausgewählt ist bestehend aus Mastdarmkrebs, Leberkrebs, Kopf- und Halskrebs, Lungenkrebs, Brustkrebs, Bauchspeicheldrüsenkrebs und Dickdarmkrebs.

2. Verfahren zur Krebserkennung nach Anspruch 1, wobei das Fragment des Polynucleotids von (a)-(d) jeweils aus den Nucleotiden 240-296 von SEQ ID NO. 1, den Nucleotiden 279-323 von SEQ ID NO. 3, den Nucleotiden 186-235 von SEQ ID NO. 5 oder den Nucleotiden 164-198 von SEQ ID NO. 7 besteht.

3. Verwendung von
(a) dem Polynucleotid mit der Nucleotidsequenz, wie in SEQ ID NO. 1 gezeigt, wobei die Zielsequenz mindestens das Nucleotidfragment der Reste 240-296 von SEQ ID NO. 1 umfasst;
(b) dem Polynucleotid mit der Nucleotidsequenz, wie in SEQ ID NO. 3 gezeigt, wobei die Zielsequenz mindestens das Nucleotidfragment der Reste 279-323 von SEQ ID NO. 3 umfasst;
(c) dem Polynucleotid mit der Nucleotidsequenz, wie in SEQ ID NO. 5 gezeigt, wobei die Zielsequenz mindestens das Nucleotidfragment der Reste 186-235 von SEQ ID NO. 5 umfasst;
(d) dem Polynucleotid mit der Nucleotidsequenz, wie in SEQ ID NO. 7 gezeigt, wobei die Zielsequenz mindestens das Nucleotidfragment der Reste 164-198 von SEQ ID NO. 7 umfasst;
und/oder
(e) einer Nucleinsäure, die zu dem Polynucleotid oder Fragment von (a)-(d) komplementär ist, bei der Herstellung eines Krebserkennungsmittels oder -kits, wobei die Nucleinsäurezielsequenz mindestens eine methylierte CpG-Stelle aufweist; und
wobei der Krebs aus der Gruppe ausgewählt ist bestehend aus Mastdarmkrebs, Leberkrebs, Kopf- und Halskrebs, Lungenkrebs, Brustkrebs, Bauchspeicheldrüsenkrebs und Dickdarmkrebs.

4. Verfahren zum Herstellen eines Krebserkennungsmittels, umfassend Bereitstellen
(a) des Polynucleotids mit der Nucleotidsequenz, wie in SEQ ID NO. 1 gezeigt, wobei die Zielsequenz mindestens das Nucleotidfragment der Reste 240-296 von SEQ ID NO. 1 umfasst;
(b) des Polynucleotids mit der Nucleotidsequenz, wie in SEQ ID NO. 3 gezeigt, wobei die Zielsequenz mindestens das Nucleotidfragment der Reste 279-323 von SEQ ID NO. 3 umfasst;
(c) des Polynucleotids mit der Nucleotidsequenz, wie in SEQ ID NO. 5 gezeigt, wobei die Zielsequenz mindestens das Nucleotidfragment der Reste 186-235 von SEQ ID NO. 5 umfasst;
(d) des Polynucleotids mit der Nucleotidsequenz, wie in SEQ ID NO. 7 gezeigt, wobei die Zielsequenz mindestens das Nucleotidfragment der Reste 164-198 von SEQ ID NO. 7 umfasst;
und/oder
(e) einer Nukleinsäure, die zu dem Polynucleotid oder Fragment von (a)-(d) komplementär ist, Konzipieren eines Erkennungsmittels zum spezifischen Erkennen einer Zielsequenz, die die volle Länge oder ein Fragment des Polynucleotids aufweist; wobei die Nucleinsäurezielsequenz mindestens eine methylierte CpG-Stelle aufweist, das Erkennungsmittel bevorzugt Folgendes umfasst: einen Primer, eine Sonde.

5. Mittel oder Kombination von Mitteln, wobei das Mittel oder die Kombination von Mitteln einen Primer umfasst, jedoch nicht darauf beschränkt ist, wobei das Mittel oder die Kombination von Mitteln spezifisch die 5-Methylierung an der CPG-Stelle/den CPG-Stellen einer Zielsequenz erkennt,
wobei die Zielsequenz die Nucleotidsequenz, wie in SEQ ID NO: 1 gezeigt, ist und die Zielsequenz das Nucleotidfragment der Reste 240-296 von SEQ ID NO: 1 umfasst und wobei der Primer wie in SEQ ID NO: 9-12 ist;
die Zielsequenz die Nucleotidsequenz, wie in SEQ ID NO: 3 gezeigt, ist und die Zielsequenz das Nucleotidfragment der Reste 279-323 von SEQ ID NO: 3 umfasst und wobei der Primer wie in SEQ ID NO: 12-15 gezeigt ist;
die Zielsequenz die Nucleotidsequenz, wie in SEQ ID NO: 5 gezeigt, ist und die Zielsequenz das Nucleotidfragment der Reste 186-235 von SEQ ID NO: 5 umfasst und wobei der Primer wie in SEQ ID NO: 12, 16-18 gezeigt ist und/oder
die Zielsequenz die Nucleotidsequenz, wie in SEQ ID NO: 7 gezeigt, ist und die Zielsequenz das Nucleotidfragment der Reste 164-198 von SEQ ID NO: 7 umfasst und wobei der Primer wie in SEQ ID NO: 12, 19-21 gezeigt ist.

6. Verwendung des Mittels oder der Kombination von Mitteln nach Anspruch 5 bei der Herstellung eines Kits zum Erkennen von Krebs, wobei der Krebs aus der Gruppe ausgewählt ist bestehend aus Mastdarmkrebs, Leberkrebs, Kopf- und Halskrebs, Lungenkrebs, Brustkrebs, Bauchspeicheldrüsenkrebs und Dickdarmkrebs.

7. Krebserkennungskit, umfassend:
einen/mehrere Behälter und das Mittel oder die Kombination von Mitteln nach Anspruch 5 in dem Behälter/den Behältern, wobei der Krebs aus der Gruppe ausgewählt ist bestehend aus Mastdarmkrebs, Leberkrebs, Kopf- und Halskrebs, Lungenkrebs, Brustkrebs, Bauchspeicheldrüsenkrebs und Dickdarmkrebs.

8. Verfahren zum Erkennen des 5-Methylierungsprofils von
(a) dem Polynucleotid mit der Nucleotidsequenz, wie in SEQ ID NO: 1 gezeigt, wobei die Zielsequenz mindestens das Nucleotidfragment der Reste 240-296 von SEQ ID NO: 1 umfasst,
(b) dem Polynucleotid mit der Nucleotidsequenz, wie in SEQ ID NO. 3 gezeigt, wobei die Zielsequenz mindestens das Nucleotidfragment der Reste 279-323 von SEQ ID NO. 3 umfasst;
(c) dem Polynucleotid mit der Nucleotidsequenz, wie in SEQ ID NO. 5 gezeigt, wobei die Zielsequenz mindestens das Nucleotidfragment der Reste 186-235 von SEQ ID NO. 5 umfasst;
(d) dem Polynucleotid mit der Nucleotidsequenz, wie in SEQ ID NO. 7 gezeigt, wobei die Zielsequenz mindestens das Nucleotidfragment der Reste 164-198 von SEQ ID NO. 7 umfasst;
und/oder
(e) einer Nukleinsäure, die zu dem Polynucleotid oder Fragment von (a)-(d) in einer Probe in Vitro komplementär ist, umfassend:
(i) Bereitstellen der Probe und Extrahieren von DNA;
(ii) Behandeln der zu erkennenden Probe, um das unmodifizierte Cytosin in Uracil umzuwandeln, Behandeln der DNA aus Schritt (i) mit Bisulfit;
(iii) Analysieren der methylierten CpG-Stellen des Polynucleotids in der durch Schritt (ii) behandelten genomischen DNA.

9. Verfahren nach Anspruch 8, wobei, in Schritt (3), die Analyseverfahren Pyrosequenzieren, Bisulfitumwandlungssequenzieren, qPCR, Zweitgenerationssequenzieren, Gesamtgenommethylierungssequenzieren, DNA-Anreicherungserkennen, vereinfachte Bisulfitsequenzierungstechnologie, HPLC oder ihre Kombination umfassen.

## Revendications

1. Procédé de détection de cancer *in vitro,* comprenant la détection du profil de 5-méthylation d'une ou de séquence(s) cible(s) comprenant :
(a) le polynucléotide ayant la séquence nucléotidique telle que présentée dans SEQ ID n° : 1, dans lequel la séquence cible comprend au moins le fragment nucléotidique des résidus 240 à 296 de SEQ ID n° : 1 ;
(b) le polynucléotide ayant la séquence nucléotidique telle que présentée dans SEQ ID n° : 3, dans lequel la séquence cible comprend au moins le fragment nucléotidique des résidus 279 à 323 de SEQ ID n° : 3 ;
(c) le polynucléotide ayant la séquence nucléotidique telle que présentée dans SEQ ID n° : 5, dans lequel la séquence cible comprend au moins le fragment nucléotidique des résidus 186 à 235 de SEQ ID n° : 5 ;
(d) le polynucléotide ayant la séquence nucléotidique telle que présentée dans SEQ ID n° : 7, dans lequel la séquence cible comprend au moins le fragment nucléotidique des résidus 164 à 198 de SEQ ID n° : 7 ;
et/ou
(e) un acide nucléique complémentaire du polynucléotide ou du fragment de (a) à (d) ;
dans lequel le cancer est sélectionné dans le groupe constitué du cancer rectal, du cancer du foie, du cancer de la tête et du cou, du cancer du poumon, du cancer du sein, du cancer du pancréas et du cancer colorectal.

2. Procédé de détection de cancer selon la revendication 1, dans lequel le fragment du polynucléotide de (a) à (d) consiste respectivement en les nucléotides 240 à 296 de SEQ ID n° : 1, les nucléotides 279 à 323 de SEQ ID n° : 3, les nucléotides 186 à 235 de SEQ ID n° : 5, ou les nucléotides 164 à 198 de SEQ ID n° : 7.

3. Utilisation
(a) du polynucléotide ayant la séquence nucléotidique telle que présentée dans SEQ ID n° : 1, dans lequel la séquence cible comprend au moins le fragment nucléotidique des résidus 240 à 296 de SEQ ID n° : 1 ;
(b) du polynucléotide ayant la séquence nucléotidique telle que présentée dans SEQ ID n° : 3, dans lequel la séquence cible comprend au moins le fragment nucléotidique des résidus 279 à 323 de SEQ ID n° : 3 ;
(c) du polynucléotide ayant la séquence nucléotidique telle que présentée dans SEQ ID n° : 5, dans lequel la séquence cible comprend au moins le fragment nucléotidique des résidus 186 à 235 de SEQ ID n° : 5 ;
(d) du polynucléotide ayant la séquence nucléotidique telle que présentée dans SEQ ID n° : 7, dans lequel la séquence cible comprend au moins le fragment nucléotidique des résidus 164 à 198 de SEQ ID n° : 7 ;
et/ou
(e) d'un acide nucléique complémentaire du polynucléotide ou du fragment de (a) à (d), dans la fabrication d'un agent ou d'un kit de détection du cancer, dans lequel la séquence cible d'acide nucléique comporte au moins un site CpG méthylé ; et
dans lequel le cancer est sélectionné dans le groupe constitué du cancer rectal, du cancer du foie, du cancer de la tête et du cou, du cancer du poumon, du cancer du sein, du cancer du pancréas et du cancer colorectal.

4. Procédé de préparation d'un agent de détection du cancer, comprenant : la fourniture
(a) du polynucléotide ayant la séquence nucléotidique telle que présentée dans SEQ ID n° : 1, dans lequel la séquence cible comprend au moins le fragment nucléotidique des résidus 240 à 296 de SEQ ID n° : 1 ;
(b) du polynucléotide ayant la séquence nucléotidique telle que présentée dans SEQ ID n° : 3, dans lequel la séquence cible comprend au moins le fragment nucléotidique des résidus 279 à 323 de SEQ ID n° : 3 ;
(c) du polynucléotide ayant la séquence nucléotidique telle que présentée dans SEQ ID n° : 5, dans lequel la séquence cible comprend au moins le fragment nucléotidique des résidus 186 à 235 de SEQ ID n° : 5 ;
(d) du polynucléotide ayant la séquence nucléotidique telle que présentée dans SEQ ID n° : 7, dans lequel la séquence cible comprend au moins le fragment nucléotidique des résidus 164 à 198 de SEQ ID n° : 7 ;
et/ou
(e) d'un acide nucléique complémentaire du polynucléotide ou du fragment de (a) à (d),
la conception d'un agent de détection pour détecter de manière spécifique une séquence cible qui est la longueur totale ou un fragment du polynucléotide ; dans lequel la séquence cible d'acide nucléique comporte au moins un site CpG méthylé ; de préférence, l'agent de détection comprend : une amorce, une sonde.

5. Agent ou combinaison d'agents, dans lequel ledit agent ou ladite combinaison d'agents inclut sans s'y limiter une amorce, dans lequel l'agent ou la combinaison d'agents détecte de manière spécifique la 5-méthylation sur le ou les sites CpG d'une séquence cible,
dans lequel la séquence cible a la séquence nucléotidique telle que présentée dans SEQ ID n° : 1, et la séquence cible comprend le fragment nucléotidique des résidus 240 à 296 de SEQ ID n° : 1, et dans lequel l'amorce est telle que présentée dans SEQ ID n° : 9 à 12 ;
la séquence cible a la séquence nucléotidique telle que présentée dans SEQ ID n° : 3, et la séquence cible comprend le fragment nucléotidique des résidus 279 à 323 de SEQ ID n° : 3, et dans lequel l'amorce est telle que présentée dans SEQ ID n° : 12 à 15 ;
la séquence cible a la séquence nucléotidique telle que présentée dans SEQ ID n° : 5, et la séquence cible comprend le fragment nucléotidique des résidus 186 à 235 de SEQ ID n° : 5, et dans lequel l'amorce est telle que présentée dans SEQ ID n° : 12, 16 à 18 ; et/ou
la séquence cible a la séquence nucléotidique telle que présentée dans SEQ ID n° : 7, et la séquence cible comprend le fragment nucléotidique des résidus 164 à 198 de SEQ ID n° : 7, et dans lequel l'amorce est telle que présentée dans SEQ ID n° : 12, 19 à 21.

6. Utilisation de l'agent ou de la combinaison d'agents selon la revendication 5 dans la fabrication d'un kit de détection de cancer, dans lequel le cancer est sélectionné dans le groupe constitué du cancer rectal, du cancer du foie, du cancer de la tête et du cou, du cancer du poumon, du cancer du sein, du cancer du pancréas et du cancer colorectal.

7. Kit de détection du cancer, comprenant :
un ou plusieurs récipient(s) et l'agent ou la combinaison d'agents selon la revendication 5 dans le ou les récipient (s), dans lequel le cancer est sélectionné dans le groupe constitué du cancer rectal, du cancer du foie, du cancer de la tête et du cou, du cancer du poumon, du cancer du sein, du cancer du pancréas et du cancer colorectal.

8. Procédé de détection du profil de 5-méthylation
(a) du polynucléotide ayant la séquence nucléotidique telle que présentée dans SEQ ID n° : 1, dans lequel la séquence cible comprend au moins le fragment nucléotidique des résidus 240 à 296 de SEQ ID n° : 1 ;
(b) du polynucléotide ayant la séquence nucléotidique telle que présentée dans SEQ ID n° : 3, dans lequel la séquence cible comprend au moins le fragment nucléotidique des résidus 279 à 323 de SEQ ID n° : 3 ;
(c) du polynucléotide ayant la séquence nucléotidique telle que présentée dans SEQ ID n° : 5, dans lequel la séquence cible comprend au moins le fragment nucléotidique des résidus 186 à 235 de SEQ ID n° : 5 ;
(d) du polynucléotide ayant la séquence nucléotidique telle que présentée dans SEQ ID n° : 7, dans lequel la séquence cible comprend au moins le fragment nucléotidique des résidus 164 à 198 de SEQ ID n° : 7 ;
et/ou
(e) d'un acide nucléique complémentaire du polynucléotide ou du fragment de (a) à (d) dans un échantillon *in vitro,* comprenant :
(i) la fourniture de l'échantillon et l'extraction de l'ADN ;
(ii) le traitement de l'échantillon à détecter pour transformer la cytosine non modifiée en uracile, le traitement de l'ADN de l'étape (i) par du bisulfite ;
(iii) l'analyse des sites CpG méthylés dudit polynucléotide dans l'ADN génomique traité par l'étape (ii).

9. Procédé selon la revendication 8, dans lequel, à l'étape (3), les méthodes d'analyse incluent : le pyroséquençage, le séquençage par transformation au bisulfite, la qPCR, le séquençage de seconde génération, le séquençage de la méthylation du génome entier, la détection par enrichissement de l'ADN, la technologie de séquençage simplifié au bisulfite, la HPLC, ou leur combinaison.
